# FASCICULE DE BREVET EUROPEEN

(11) **EP 0 644 170 B1**
(45) Date de publication et mention de la délivrance du brevet: **13.10.1999**
(21) Numéro de dépôt: 94401914.0
(22) Date de dépôt: 30.08.1994
(51) Int. Cl.: C07C 17/20, C07C 19/08

(54) **Fabrication des bromures de perfluoroalkyle solides**
Herstellung von festen Perfluoralkylbromiden
Preparation of solid perfluoroalkylbromides

(30) Priorité: 16.09.1993 FR 9311045
(43) Date de publication de la demande: 22.03.1995
(73) Titulaire: ELF ATOCHEM S.A., 92800 Puteaux, Hauts-de-Seine (FR)
(72) Inventeur: Drivon, Gilles, F-69850 Saint Martin en Haut (FR); Bindi, Dominique, F-69110 Sainte-Foy-Les-Lyon (FR)
(74) Mandataire: Leboulenger, Jean

(56) Documents cités:
- EP-A- 0 450 584
- EP-A- 0 515 258
- DE-A- 4 116 361

## Description

La présente invention concerne le domaine des bromures de perfluoroalkyle et a plus particulièrement pour objet la fabrication des bromures de perfluoroalkyle solides à température ambiante, c'est-à-dire ceux dont le radical perfluoroalkyle CₙF₂ₙ₊₁, linéaire ou ramifié, contient 10 ou plus de 10 atomes de carbone.

En raison de leur développement prometteur dans le domaine médical comme radiopaques (agents de contraste aux rayons X) ou comme transporteurs d'oxygène dans les substituts du sang, les bromures de perfluoroalkyle ont fait l'objet, ces dernières années, de nombreux brevets concernant leur synthèse à partir des sulfochlorures de perfluoroalkyle R_{F}SO₂Cl (EP 0298870 et 0429331), des iodures de perfluoroalkyle R_{F}I (JP 85-184033, EP 0428039, EP 0450584, EP 0515258 et EP 0519808) ou des hydrogénoperfluoroalcanes R_{F}H (US 3456024 et EP 0549387), ainsi que leur purification (EP 0513783). Les procédés décrits dans ces brevets visent essentiellement la fabrication de bromures de perfluoroalkyle liquides à température ambiante et en particulier celle du bromure de n.perfluorooctyle C₈F₁₇Br (connu sous l'abréviation PFOB).

Parmi ces procédés connus, la voie la plus directe d'obtention des bromures de perfluoroalkyle est évidemment celle décrite dans les brevets EP 0450584 et 0515258 qui consiste en la bromation thermique en phase gazeuse des iodures correspondants R_{F}I qui sont disponibles en quantités industrielles. En aval du réacteur, une installation industrielle pour la fabrication d'un bromure de perfluoroalkyle doit comprendre divers appareillages permettant de récupérer l'iode sous-produit et d'isoler le bromure de perfluoroalkyle, au moyen d'opérations unitaires telles que réduction de l'iode, neutralisation, décantation, neutralisation-lavage, etc...

Lorsque le bromure de perfluoroalkyle désiré est liquide à température ambiante, notamment le PFOB dont le point de fusion est 6°C, ces divers appareillages sont des matériels très simples et peu coûteux car ils n'ont pas besoin d'être tracés ou de posséder une double enveloppe de chauffage.

Une installation industrielle conçue avec de tels matériels pour la fabrication d'un bromure liquide comme le PFOB ne peut pas être utilisée, sans modification et investissement important, pour fabriquer un bromure solide tel que le bromure de perfluorodécyle (ci-après PFDB) dont le point de fusion est 55°C, l'iodure de perfluorodécyle de départ ayant un point de fusion de 65°C.

Il a maintenant été trouvé que ce problème peut être résolu en effectuant la bromation thermique d'un iodure de perfluoroalkyle conduisant à un bromure normalement solide en présence d'un bromure de perfluoroalkyle normalement liquide. Ainsi, lors des différentes opérations de purification et jusqu'à l'étape finale de distillation, le bromure normalement solide se trouve sous une forme liquide véhiculable à température ambiante.

Le procédé selon l'invention pour la fabrication d'un bromure de perfluoroalkyle normalement solide par bromation thermique en phase gazeuse d'un iodure de perfluoroalkyle contenant 10 ou plus de 10 atomes de carbone est donc caractérisé en ce qu'on opère en présence d'un bromure de perfluoroalkyle normalement liquide.

Dans les expressions "normalement solide" et "normalement liquide", le terme "normalement" s'entend pour la température ambiante à la pression atmosphérique.

Comme bromure de perfluoroalkyle normalement liquide, on peut utiliser tout bromure contenant de 4 à 8 atomes de carbone, mais industriellement on préfère employer le bromure de perfluorohexyle et, plus particulièrement, le bromure de perfluorooctyle.

La quantité de bromure de perfluoroalkyle normalement liquide à mettre en oeuvre peut varier dans de larges limites et dépend essentiellement de la solubilité respective du bromure liquide utilisé et du bromure solide à produire. Cette quantité doit, conformément à l'invention, être suffisante pour maintenir en solution le bromure à produire, durant toutes les étapes de post-traitement (réduction de l'iode, décantation, lavage,...). A titre purement indicatif, pour la fabrication du PFDB à partir du iodure de perfluorodécyle en présence de PFOB, le rapport pondéral initial C₁₀F₂₁I/PFOB pourra aller de 0,01 à 1,5, de préférence entre 0,1 et 1.

La réaction de bromation du iodure de perfluoroalkyle solide contenant 10 ou plus de 10 atomes de carbone (ci-après R_{F}I) peut, comme dans la technique antérieure, être effectuée dans un réacteur tubulaire à une température allant de 200 à 550°C, de préférence comprise entre 350 et 450°C. Le brome et une solution de R_{F}I dans le bromure liquide peuvent être alimentés séparément au réacteur. Il est cependant préférable d'alimenter le réacteur avec une solution unique de brome et de R_{F}I dans le bromure de perfluoroalkyle normalement liquide ; ceci permet d'opérer avec une seule alimentation, d'assurer un rapport molaire Br₂/R_{F}I constant et, en cas d'arrêt accidentel ou de dérèglement de la pompe d'alimentation, d'éviter tout risque de formation de perfluoroisobutylène très toxique.

Le rapport molaire Br₂/R_{F}I peut aller de 0,4 à 2, mais est de préférence compris entre 0,5 et 0,8.

La réaction du brome avec le R_{F}I en phase gazeuse étant très rapide, le temps de contact, c'est-à-dire le temps de séjour des réactifs dans le réacteur, n'est pas un paramètre critique. Un temps de contact compris entre 1 seconde et 2 minutes convient généralement bien, mais industriellement on préfère opérer avec un temps de contact allant de 5 à 60 secondes.

Le réacteur peut être un tube vide en verre, quartz ou Inconel 600, mais il peut éventuellement contenir un substrat solide inerte (par exemple verre ou quartz) pour faciliter le contact entre les gaz. Bien que cela ne soit pas indispensable, on peut aussi opérer en présence d'un gaz inerte, par exemple l'azote.

Industriellement on préfère effectuer la bromation à la pression atmosphérique, mais on ne sortirait pas du cadre de la présente invention en travaillant à une pression légèrement inférieure ou supérieure à la pression atmosphérique pourvu que le système réactionnel reste à l'état gazeux.

En sortie du réacteur et après refroidissement jusqu'à une température comprise entre environ 150 et 250°C, les gaz sont traités à une température inférieure à 40°C par une solution aqueuse d'un agent réducteur tel que, par exemple, le sulfite de sodium, en une quantité suffisante pour réduire l'iode formé et le brome éventuellement présent (Br₂ non réagi). Après décantation à température ambiante, la phase organique inférieure est neutralisée par une solution aqueuse d'un agent alcalin quelconque, puis décantée à nouveau avant distillation à pression atmosphérique ou sous pression réduite.

Les exemples suivants illustrent l'invention sans la limiter.

### EXEMPLE 1

On prépare un mélange homogène composé de 3021 g de bromure de perfluorooctyle C₈F₁₇Br, 3021 g de iodure de perfluorodécyle C₁₀F₂₁I et 434 g de brome, et on le maintient à une température comprise entre 25 et 40°C.

Ce mélange est ensuite introduit en 2 heures dans un réacteur Pyrex de 3 litres fonctionnant dans les conditions opératoires suivantes:
- température : 400 ± 10°C
- temps de contact: environ 30 secondes
- rapport molaire Br₂/C₁₀F₂₁I = 0,58

Après refroidissement, les gaz sortant du réacteur sont traités par 2667 g d'une solution aqueuse de sulfite de sodium à 18 % à 35-40°C. Après décantation à température ambiante, on obtient deux phases:
- une phase aqueuse supérieure qui est dosée par argentimétrie pour déterminer sa teneur en ions bromure et iodure (Br⁻ = 0,78 éq. et I⁻ = 4,62 éq.)
- une phase organique inférieure qui, après lavage à 20°C au moyen de 400 g d'une solution aqueuse 2N d'hydroxyde de sodium, pèse 5808 g et dont l'analyse par chromatographie en phase gazeuse sur un appareil HEWLETT PACKARD 5890 (détecteur FID; colonne DB1 capillaire 60 m) donne la composition pondérale suivante:

| | |
|---|---|
| C₈F₁₇Br | 50,54 % |
| C₁₀F₂₁Br | 48,63 % |
| C₁₀F₂₁I | 0,37 % |

ce qui correspond à un taux de transformation du C₁₀F₂₁I supérieur à 99 % et à une sélectivité en C₁₀F₂₁Br supérieure à 99,5 %.

Après distillation de la phase organique sur une colonne KERAPAK de 17 plateaux, on obtient 2240 g de bromure de perfluorodécyle (pureté ≥ 99,9 %).

### EXEMPLE 2

On opère comme à l'exemple 1 à partir d'un mélange composé de 4903 g de C₈F₁₇Br, 1226 g de C₁₀F₂₁I et 187 g de brome, soit un rapport molaire Br₂/C₁₀F₂₁I de 0,62. On obtient les résultats suivants:

### Phase aqueuse

| | |
|---|---|
| Br⁻ | = 0,46 éq. |
| I⁻ | = 1,89 éq. |

### Phase organique (6003 g)

| | |
|---|---|
| C₈F₁₇Br | 80,10 % |
| C₁₀F₂₁Br | 19,30 % |
| C₁₀F₂₁I | 0,06 % |

ce qui correspond à un taux de transformation du C₁₀F₂₁I supérieur à 99 % et à une sélectivité en C₁₀F₂₁Br supérieure à 99,5 %.

### EXEMPLE 3

On opère comme à l'exemple 1, mais en remplaçant le bromure de perfluorooctyle par le bromure de perfluorohexyle C₆F₁₃Br. On utilise un mélange composé de 2653 g de C₆F₁₃Br, 2653 g de C₁₀F₂₁I et 390 g de brome, soit un rapport molaire Br₂/C₁₀F₂₁I égal à 0,59.

L'analyse des phases aqueuse et organique donne les résultats suivants:

### Phase aqueuse

| | |
|---|---|
| Br⁻ | = 0,77 éq. |
| I⁻ | = 4,07 éq. |

### Phase organique (5088 g)

| | |
|---|---|
| C6F₁₃Br | 51,30 % |
| C₁₀F₂₁Br | 48,10 % |
| C₁₀F₂₁I | 0,25 % |

Comme dans les exemples 1 et 2, le taux de transformation du C₁₀F₂₁I est supérieur à 99 % et la sélectivité en C₁₀F₂₁Br est supérieure à 99,5 %.

## Revendications

1. Procédé de fabrication d'un bromure de perfluoroalkyle normalement solide par bromation thermique en phase gazeuse d'un iodure de perfluoroalkyle contenant 10 ou plus de 10 atomes de carbone, caractérisé en ce qu'on opère en présence d'un bromure de perfluoroalkyle normalement liquide en une quantité suffisante pour que le mélange des deux bromures soit liquide à température ambiante.

2. Procédé selon la revendication 1, dans lequel le bromure de perfluoroalkyle normalement liquide est le bromure de perfluorohexyle ou le bromure de perfluorooctyle.

3. Procédé selon la revendication 1 ou 2, dans lequel le rapport molaire Br₂/iodure de perfluoroalkyle est compris entre 0,4 et 2, de préférence entre 0,5 et 0,8.

4. Procédé selon l'une des revendications 1 à 3, dans lequel on alimente le réacteur avec un mélange homogène composé de brome, du iodure de perfluoroalkyle et du bromure de perfluoroalkyle normalement liquide.

5. Procédé selon l'une des revendications 1 à 4 pour la fabrication du bromure de perfluorodécyle à partir du iodure de perfluorodécyle C₁₀F₂₁I en présence de bromure de perfluorooctyle C₈F₁₇Br, dans lequel le rapport pondéral initial C₁₀F₂₁I/C₈F₁₇Br est compris entre 0,01 et 1,5, de préférence entre 0,1 et 1.

## Claims

1. Process for the manufacture of a normally solid perfluoroalkyl bromide by thermal-bromination in the gas phase of a perfluoroalkyl iodide containing 10 or more than 10 carbon atoms, characterized in that the bromination is carried out in the presence of a normally liquid perfluoroalkyl bromide in a quantity sufficient for the mixture of the two bromides to be liquid at room temperature.

2. Process according to Claim 1, in which the normally liquid perfluoroalkyl bromide is perfluorohexyl bromide or perfluorooctyl bromide.

3. Process according to Claim 1 or 2, in which the Br₂/perfluoroalkyl iodide molar ratio is between 0.4 and 2, preferably between 0.5 and 0.8.

4. Process according to one of Claims 1 to 3, in which the reactor is supplied with a homogeneous mixture composed of bromine, the perfluoroalkyl iodide and the normally liquid perfluoroalkyl bromide.

5. Process according to one of Claims 1 to 4 for the manufacture of perfluorodecyl bromide from perfluorodecyl iodide C₁₀F₂₁I in the presence of perfluorooctyl bromide C₈F₁₇Br, in which the initial C₁₀F₂₁I/C₈F₁₇Br ratio by weight is between 0.01 and 1.5, preferably between 0.1 and 1.

## Patentansprüche

1. Verfahren zur Herstellung eines normalerweise festen Perfluoralkylbromids durch thermische Bromierung von Perfluoralkyliodid mit 10 oder mehr als 10 Kohlenstoffatomen in der Gasphase, dadurch gekennzeichnet, daß man in Anwesenheit eines sich normalerweise in flüssiger Phase befindlichen Perfluoralkylbromids in ausreichender Menge arbeitet, damit das Gemisch der beiden Bromide bei Raumtemperatur flüssig ist.

2. Verfahren nach Anspruch 1, bei dem das normalerweise flüssige Perfluoralkylbromid Perfluorhexyl- oder Perfluoroctylbromid ist.

3. Verfahren nach Anspruch 1 oder 2, bei dem das Molverhältnis Br₂/Perfluoralkyliodid zwischen 0,4 und 2, vorzugsweise zwischen 0,5 und 0,8, liegt.

4. Verfahren nach einem der Ansprüche 1 bis 3, bei dem man den Reaktor mit einem homogenen Gemisch aus Brom, Perfluoralkyliodid und normalerweise flüssigem Perfluoralkylbromid speist.

5. Verfahren nach einem der Ansprüche 1 bis 4 Zur Herstellung von Perfluordecylbromid aus Perfluorderyliodid C₁₀F₂₁I in Gegenwart von Perfluoroctylbromid C₈F₁₇Br, wobei das Ausgangsgewichtsvehältnis C₁₀F₂₁I/C₈F₁₇Br zwischen 0,01 und 1,5, vorzugsweise zwischen 0,1 und 1, liegt.
